# EUROPEAN PATENT APPLICATION

(11) **EP 0 848 958 A2**
(43) Date of publication of application: **24.06.1998**
(21) Application number: 97308949.3
(22) Date of filing: 07.11.1997
(51) Int. Cl.: A61L 2/06, B01J 3/03

(54) **Autoclaves and methods of manufacture**

(30) Priority: 18.12.1996 GB 9626222
(71) Applicant: Smiths Industries Public Limited Company, London, NW11 8DS (GB)
(72) Inventor: Clayton, Patrick Brian, Worthing, West Sussex BN11 5HT (GB)
(74) Representative: Flint, Jonathan McNeill

(57) **Abstract**

An autoclave sterilizer has a chamber 1 with a gas outlet 10 and a gas inlet 11. The gas outlet 10 is connected to an atmosphere vent 14 and to the inlet of a pump 22 via a valves 15 and 28. The inlet of the pump 22 is also connected to an atmosphere inlet 26 via a valve 23. The outlet of the pump 22 is connected to the chamber gas inlet 11 and to a second atmosphere vent 32 via valves 18, 21 and 33. A second atmosphere inlet 34 connects with the chamber gas inlet 11. After sterilization, the pump 22 draws a vacuum in the chamber 1 and air is then admitted via the atmosphere inlet 34. The pump 22 then pumps air from the atmosphere inlet 26 to the vent 32, bypassing the chamber 1, to cool the pump.

## Description

This invention relates to autoclaves of the kind including a pump, the autoclave being arranged to pump gas from the autoclave chamber at the end of a treatment cycle.

Autoclaves, such as for sterilization, subject articles within the autoclave chamber to elevated pressure and temperature in the presence of a vapour, which is usually steam. The autoclave may have a vacuum pump for pumping out air before the sterilization or other treatment is started. Whereas larger autoclaves generally have a pump capable of operating at high temperature and levels of humidity, such as a liquid ring pump, it is more difficult to provide suitable pumps in smaller, lower cost autoclaves. Another problem with autoclaves arises from the fact that, at the end of the sterilization or other treatment, the articles are usually wet, especially if the articles are porous.

It is an object of the present invention to provide an improved autoclave and method of operation of an autoclave.

According to one aspect of the present invention there is provided an autoclave of the above-specified kind, characterised in that the autoclave is arranged subsequently to pump air from outside the chamber through the pump.

The autoclave is preferably operable to pump air from outside the chamber through the pump to atmosphere without passing through the chamber and may be operable to pump air from outside the chamber through the pump to the chamber. A heater may be located between the pump and the chamber. A chamber gas outlet is preferably connected to an atmosphere vent via a first electrically-operated valve, a chamber gas inlet being connected to an atmosphere inlet via a second electrically-operated valve. The inlet of the pump may be connected with both the chamber gas outlet and an atmosphere inlet via respective valves. The outlet of the pump is preferably connected to both the chamber gas inlet and to an atmosphere vent via respective valves.

According to another aspect of the present invention there is provided an autoclave having a chamber with a gas outlet and a gas inlet, a first pipe extending from the gas outlet to an atmosphere vent via an electrically-operated valve, a second pipe extending from the gas inlet to an atmosphere inlet via a pump, characterised in that a third pipe connects the first pipe with an inlet of the pump, and that a fourth pipe connects an outlet of the pump to an atmosphere vent.

According to a further aspect of the present invention there is provided a method of operating an autoclave including the steps of subjecting articles in the autoclave chamber to treatment with steam at elevated temperature and pressure, pumping steam out of the chamber with a pump and subsequently cooling the pump by pumping clean, sterile air from outside the chamber through the pump.

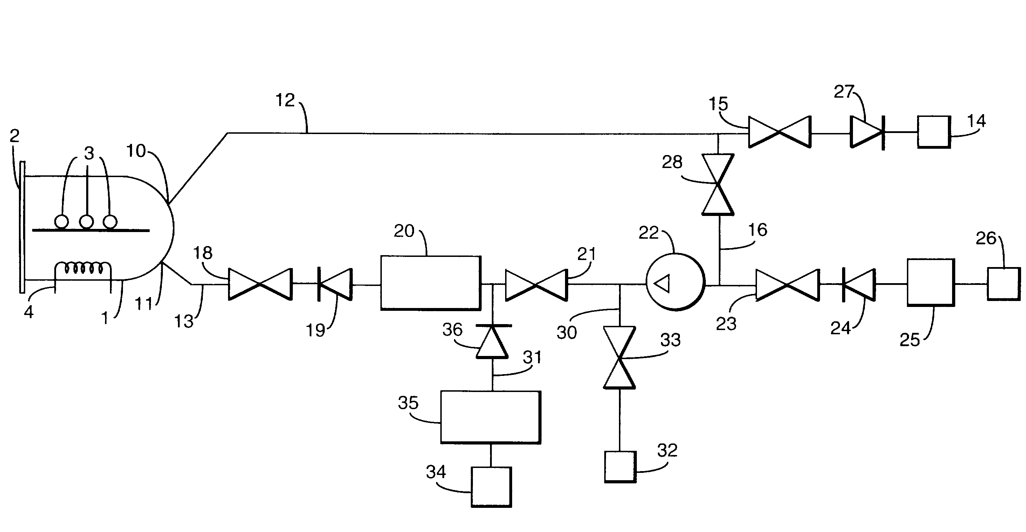

An autoclave sterilizer according to the present invention, will now be described, by way of example, with reference to the accompanying drawing, which shows the sterilizer schematically.

The sterilizer has a chamber 1 with a door 2, and contains articles 3 to be sterilized. The chamber 1 has a heater 4 by which water in the chamber can be heated to generate steam at elevated temperature and pressure, in the usual way. The chamber has two gas openings 10 and 1 connected to respective pipes 12 and 13. One pipe 12 extends to an atmosphere vent 14 via an electrically-operated valve 15 and a one-way valve 27.

The other pipe 13 includes the following components, in order, from the chamber: a second electrically-operated valve 18, a one-way valve 19, a heater 20, a third electrically-operated valve 21, a pump 22, a fourth electrically-operated valve 23, a second one-way valve 24, an air filter 25 and an atmosphere inlet 26. The pump 22 can be a relatively low-performance, low-cost pump of the kind that would not normally be suitable for use in prolonged operation at high temperature and humidity levels. The filter 25 is a bacteria and viral filter effective at removing any bacteria and virus in gas passing through the filter, such that the gas that does pass through is clean and sterile.

A first branch pipe 16 including a fifth electrically-operated valve 28 is connected at one end to the pipe 12, between the chamber 1 and the valve 15. The other end of the branch pipe 16 is connected to the pipe 13, between the pump 22 and the valve 23. Two further branch pipes 30 and 31 are also connected to the pipe 13. One branch pipe 30 is connected to the pipe 13 at a location between the pump 22 and the third electrically-operated valve 21 and connects to a third atmosphere vent 32 via a sixth electrically-operated valve 33. The other branch pipe 31 is connected to the pipe 13 between the heater 20 and the third valve 21 and connects to an atmosphere inlet 34 via a filter 35 and a one-way valve 36.

In operation, after the articles 3 have been placed in the chamber 1 and the door 2 has been closed, the pump 22 is turned on and the valves 28 and 33 are opened. At this time, the other valves 15, 18, 21 and 23 are held closed. The pump 22, therefore, pumps air out of the chamber 1, via pipe 12 and the branch pipes 16 and 30, to the vent 32. The valves 28 and 33 are then closed and the heater 4 is turned on to heat water in the chamber 1 to produce the steam at elevated temperature and pressure needed to sterilize the articles 3.

When sterilization has been completed, the valve 15 is opened and steam is exhausted to atmosphere via the non-return valve 27 and the vent 14. When excess pressure in the chamber 1 has been released, the valve 15 is closed and the pump 22 is turned on again. The valves 28 and 33 are opened so that any residual steam in the chamber 1 is exhausted to atmosphere via the vent 32 and the pressure is reduced below atmosphere. The valve 28 is then closed and valve 18 is opened to allow clean, sterile, dry air to enter the chamber 1 via the filter 35, one-way valves 36 and 19, and the heater 20. The heater 20 heats the air admitted to the chamber 1, in order to promote drying. In this way, the steam is replaced with clean, dry, warm air. While air is being admitted to the chamber 1, the valves 23 and 33 are opened and the pump 22 is run so that cool air from the inlet 26 circulates through the pump, after being filtered by the filter 25. This helps cool the pump 22 and its associated piping.

In the next phase of drying, the valve 33 is closed and the valve 21 is opened so that the pump 22 pumps air from the inlet 26 into the chamber 1 via the heater 20. This may be allowed to raise the pressure in the chamber 1 above atmosphere or it may be vented to atmosphere by opening the valve 15 so that gas can escape via the opening 10, the pipe 12 and the vent 14.

Where the pump is used to raise the pressure in the chamber 1, the sterilizer subsequently vents the chamber before the door can be opened. Alternatively, the sterilizer could pump down the chamber 1 again and then admit filtered air.

Following the drying phase, the heater 20 could be turned off and the pump 22 used to pump cool, filtered air through the chamber in order to cool the chamber 1 and its contents.

Because the pump 22 is used to pump air from outside the chamber, after pumping out the hot steam from within the chamber, this helps cool the pump and prevent damage. In this way, the sterilizer can employ a pump not specifically designed for prolonged operation at high temperature and humidity, thereby enabling it to be less bulky and heavy, and of lower cost than would otherwise be the case.

## Claims

1. An autoclave including a pump (22), the autoclave being arranged to pump gas from the autoclave chamber (1) at the end of a treatment cycle, characterised in that the autoclave is arranged subsequently to pump air from outside the chamber (1) through the pump (22).

2. An autoclave according to Claim 1, characterised in that the autoclave is operable to pump air from outside the chamber (1) ) through the pump (22) to atmosphere without passing through the chamber (1).

3. An autoclave according to Claim 1 or 2, characterised in that the autoclave is operable to pump air from outside the chamber (1) ) through the pump (22) to the chamber (1).

4. An autoclave according to Claim 3, including a heater (20) located between the pump (22) and the chamber (1).

5. An autoclave according to any one of the preceding claims, characterised in that a chamber gas outlet (10) is connected to an atmosphere vent (14) via a first electrically-operated valve (15), and that a chamber gas inlet (11) is connected to an atmosphere inlet (34) via a second electrically-operated valve (18).

6. An autoclave according to Claim 5, characterised in that the inlet of the pump (22) is connected with both the chamber gas outlet (10) and an atmosphere inlet (26) via respective valves (28 and 23).

7. An autoclave according to Claim 5 or 6, characterised in that the outlet of the pump (22) is connected to both the chamber gas inlet (11) and to an atmosphere vent (32) via respective valves (21, 18 and 33).

8. An autoclave having a chamber (1) with a gas outlet (10) and a gas inlet (11), a first pipe (12) extending from the gas outlet (10) to an atmosphere vent (14) via an electrically-operated valve (15), a second pipe (13) extending from the gas inlet (11) to an atmosphere inlet (26) via a pump (22), characterised in that a third pipe (16) connects the first pipe (12) with an inlet of the pump (22), and that a fourth pipe (30) connects an outlet of the pump (22) to an atmosphere vent (32).

9. A method of operating an autoclave including the steps of subjecting articles (3) in the autoclave chamber (1) to treatment with steam at elevated temperature and pressure, pumping steam out of the chamber (1) with a pump (22) and subsequently cooling the pump (22) by pumping clean, sterile air from outside the chamber (1) through the pump (22).

10. A method according to Claim 9, characterised in that the pump (22) is cooled by pumping clean, sterile air from outside the chamber (1) through the pump (22) to atmosphere without passing through the chamber (1).
